# EUROPÄISCHE PATENTSCHRIFT

(11) **EP 3 641 670 B1**
(45) Veröffentlichungstag und Bekanntmachung des Hinweises auf die Patenterteilung: **26.04.2023**
(21) Anmeldenummer: 19704543.8
(22) Anmeldetag: 31.01.2019
(51) Int. Cl.: A61B 17/16, A61B 17/17, A61B 17/02, A61B 17/34, A61B 17/00, A61B 17/90

(54) **INSTRUMENTEN-SET FÜR WIRBELSÄULENOPERATIONEN**
INSTRUMENT SET FOR SPINAL OPERATIONS
KIT D'INSTRUMENTS POUR DES OPÉRATIONS RACHIDIENNES

(30) Priorität: 21.03.2018 DE 102018002356; 14.08.2018 DE 102018006442
(43) Veröffentlichungstag der Anmeldung: 29.04.2020
(73) Patentinhaber: Joimax GmbH, 76227 Karlsruhe (DE)
(72) Erfinder: RIES, Wolfgang, 76351 Linkenheim (DE)
(74) Vertreter: Lichti - Patentanwälte Partnerschaft mbB
(86) Internationale Anmeldenummer: PCT/EP2019/000030
(87) Internationale Veröffentlichungsnummer: WO 2019/179653

(56) Entgegenhaltungen:
- WO-A1-2017/119401
- DE-A1- 4 328 690
- DE-U1-202005 016 762
- US-A1- 2007 066 977
- US-A1- 2017 021 147
- US-A1- 2017 135 704
- US-B1- 9 028 522

## Beschreibung

Die Erfindung betrifft ein Instrumenten-Set für Wirbelsäulenoperationen.

Bei dem Einführen von Operationsinstrumenten zu einem Operationsort in einen - menschlichen - Körper trifft, auch unter Röntgensicht, insbesondere wenn es sich um ein enges Operationsgebiet handelt, der Operateur dieses manchmal nicht sofort. Es ist dann notwendig, die eingeführten Instrumente teilweise oder vollständig zurückzuziehen und erneut möglichst zum richtigen Operationsort einzuführen, was nicht nur aufwendig, sondern auch für die Patienten belastend ist.

Dies gilt beispielsweise für das Entfernen von Osteophyten, also Knochenwucherungen, insbesondere an Wirbeln der Wirbelsäule, die auf Nerven drücken und damit zu erheblichen Schmerzen eines Patienten führen können oder aber, wenn sie an Frontseiten von Wirbelvorsprüngen (englisch: processes) auftreten, die Beweglichkeit einschränken können.

Die US 2007/0066977 A1 zeigt ein Wechselsystem zum Vergrößern des Querschnittsbereichs eines Zugangswegs durch weiches Körpergewebe zur Oberfläche eines Knochens mit einem länglichen Wechselstab und einer ebenfalls länglichen Wechselkanüle mit einem sich durch diese erstreckenden zentralen Hohlraum mit einem abgeschrägten distalen Ende, wobei der Wechselstab axial im Hohlraum der Wechselkanüle gleiten kann. Zum einen ist der Querschnitt des Systems nicht vorzugsweise groß, zum anderen muss auch bei einer geringen Fehlpositionierung das System wieder entfernt und erneut eingeführt werden.

Die WO 2007/119401 A1 offenbart ein Operationssystem, das eine Änderung der Perspektive eines endoskopischen Bildes auf einem Monitor unterdrücken soll, wenn sich ein Überrohr gedreht hat. Das System umfasst hierzu ein Überrohr, das ein Behandlungswerkzeug und ein Endoskop so hält, dass diese entlang der Längsachse bewegbar sind. Darüber hinaus weist das Überrohr ein Endoskophalteteil mit einer Halteoberfläche auf, das die Rotation des Endoskops um seine Zentralachse erlaubt. Weiter ist ein Halteteil für das Arbeitswerkzeug vorgesehen. Teil des Systems ist weiterhin das Endoskop selbst, das in das Überrohr einsetzbar ist und ein Ausrichtungserhaltungsteil, das die Umfangsausrichtung des Endoskops selbst dann beibehält, indem sich das Überrohr um seine Längsachse dreht.

Für eine rasche und präzise Durchführung von Operationen zur Verblockung von Wirbelkörpern zeigt die DE 43 28 690 A1 ein Implantationssystem, bei dem die Bearbeitungsinstrumente, wie Bohrer, Meißel, Knochenräumwerkzeug, Implantateinführwerkzeug, mit einer Hilfseinrichtung zusammenwirken, die ein genaues und rasches Ansetzen des jeweiligen Werkzeugs ermöglicht. Die Hilfseinrichtung kann ein langgestrecktes Führungsinstrument sein, das aus Führungsschienen oder einem Führungszylinder besteht und eine Spreizvorrichtung hat. Ein Distanzhalter, der nach dem Spreizen der betreffenden Wirbelkörper in den Spalt eingesetzt wird, kann auch als Hilfseinrichtung ausgebildet werden.

Die DE 20 2005 016 762 U1 zeigt einen Facettengelenkfräser mit einem Schaft und einer Zähnung an der distalen Stirnseite, die gegenüber dem Schaftdurchmesser aufgeweitet ist.

Die US 9,028,522 B1 zeigt eine Vorrichtung und ein Verfahren zur chirurgischen Dilatation und Retraktion von Gewebe während eines medizinischen Eingriffs, beispielsweise einer Wirbelsäulenoperation. Die Vorrichtung umfasst eine Vielzahl von gleitend ineinander eingreifenden Dilatoren. Mindestens einer der Dilatoren kann einen exzentrisch positionierten kreisförmigen Kanal, eine allgemein längliche Au-ßenwandform und einen Längsspalt in einer Wand aufweisen. Hierdurch soll eine außermittige Dilatation ermöglicht werden.

Die Dilatoren weisen plane achssenkrechte distale Stirnseiten auf. Dilatoren dienen dazu möglichst wenig traumatisierend Weichgewebe aufzuweiten, damit letztendlich über sie ein Arbeitsrohr geschoben werden kann, durch welches nach Entfernen der Dilatoren Arbeitsinstrumente zu einem Arbeitsort, beispielsweise bei einer Bandscheibe einer Wirbelsäule eingeführt werden können, um durch die Wirbelsäule führende Nerven zumindest bedrängende Teile der Bandscheibe zu entfernen. Aufgrund des Umstandes, dass die distalen Stirnseiten der Dilatoren plan senkrecht zu deren Achse sind, wird der Führungsweg durch den ursprünglich eingeführten Führungsdraht bestimmt. Die nicht zylindrischen Dilatoren mögen zwar vor dem Einführen angular in einer gewünschten Position positioniert werden; sie sind nach dem Einführen aber in ihrer Radial- und/oder Angularposition nicht veränderbar.

Die US 2017/0021147 A1 zeigt ein System zum Bereitstellen eines Zugangs zu einer Operationsstelle mit mehreren Dilatoren, die miteinander koppelbar sind. Bis auf den ersten oder innersten Dilator weisen die weiteren Dilatoren einen seitlichen Schlitz auf. Auch hier sind die Stirnseiten der Dilatoren weitgehend eben ausgebildet. Im Übrigen gilt das gleiche wie das zur US 9,028,522 B1 Gesagte. Insbesondere weisen die Dilatoren keine exzentrisch zu ihrem Hohlraum ausgebildete distale Zunge auf und ist auch über die Dilatoren hinaus kein weiterer einen Hohlraum aufweisender Führungsstab mit einer exzentrisch zu seinem Hohlraum ausgebildeten Spitze, um die er verschwenkbar ist, vorhanden.

Es ist daher Aufgabe der Erfindung, ein Instrumenten-Set zu schaffen, mittels dessen im Rahmen eines Operationsablaufs auch eine Korrektur des Operationsortes, also des Gebiets, an dem sich die distalen Enden von Instrumenten eines Instrumenten-Sets befinden, möglich ist, ohne dass die Instrumente aus dem Körper herausgezogen werden müssen.

Die Aufgabe wird mit einem erfindungsgemäßen Instrumenten-Set nach dem Anspruch 1 gelöst.

Insbesondere durch das Merkmal, dass die Breite der Schlitzöffnung kleiner ist als der Durchmesser der Nut, wird verhindert, dass bei gegebener Schlitzöffnung ein axial in den so als Nut ausgebildeten Hohlraum eingeführtes Instrument, wie beispielsweise ein Führungsrohr, sich aus der Nut des Führungsstabs seitlich herausbewegt.

In Weiterbildung ist vorgesehen, dass der Hohlraum exzentrisch zur Außenkontur und/oder zur Symmetrieachse der Außenkontur des Führungsstabs ausgebildet ist. Darüber hinaus sieht die Erfindung in bevorzugter Ausgestaltung vor, dass die distale Zunge des Führungsstabs exzentrisch zu einer Symmetrieachse des Führungsstabs angeordnet ist.

Weitere Ausgestaltungen sehen vor, dass die die Nut umgebende Innenwandfläche des Führungsstabs teilzylindermantelförmig ist und/oder dass die Außenwandfläche des Führungsstabs teilzylindermantelförmig ist. Hierdurch wird die Kompatibilität des Führungsstabs mit anderen Instrumenten, die in ihn eingeführt werden, oder aber in die er eingeführt wird bzw. die über ihn hin eingeführt werden, sichergestellt.

In weiteren bevorzugten Ausgestaltungen ist vorgesehen, dass die Schlitzöffnung der Nut des Führungsstabs der Zunge diagonal gegenüber liegt und/oder dass Querabmessungen von Stilett und/oder Führungsdraht an das Lumen des Hohlraums der Hohlnadel angepasst sind.

Eine äußerst bevorzugte Weiterbildung des erfindungsgemäßen Instrumenten-Sets ist gekennzeichnet durch eine Arbeitshülse mit einem sich längserstreckenden, insbesondere zylindrischen Hohlraum, der einen dem Querschnitt des Führungsrohrs, vorzugsweise aber dem Querschnitt des Führungsstabs mindestens gleichen Querschnitt aufweist.

Die Arbeitshülse weist dabei in bevorzugter Ausgestaltung an seiner distalen Stirnseite eine Zahnreihe auf, die sich über einen Teil des Umfangs der Arbeitshülse, vorzugsweise über die Hälfte deren Umfangs erstreckt.

Die Zähne der Zahnreihe sind insbesondere asymmetrisch ausgebildet, wobei eine erste Flanke der Zähne als flaches zur distalen Spitze eines Zahns hin geneigtes S ausgebildet ist, während dass eine zweite Flanke achsparallel ausgerichtet ist.

Gegenüber der Zahnreihe erstreckt sich vorzugsweise über einen Teil des Umfangs der Arbeitshülse eine Lippe mit durchgehender distaler Stirnseite. Insbesondere liegen die Spitzen der Zähne und die Stirnseite der Lippe auf gleicher axialer Höhe.

Das Instrumenten-Operationsset kann weitergebildet sein durch eine Hohlnadel mit einem Stilett und/oder einem Führungsdraht, wobei vorzugsweise Querabmessungen von Stilett und/oder Führungsdraht an das Lumen des Hohlraums der Hohlnadel angepasst sind und/oder durch Dilatoren mit an Führungsrohr und/oder Führungsstab oder aber aneinander angepassten Lumen darüber hinaus ist das Operationsset weitergebildet durch Arbeitswerkzeuge wie Fräser und Meißel, deren radiale Abmessungen insbesondere an das Lumen der Arbeitshülse angepasst sind.

Der Einsatz des erfindungsgemäßen Instrumenten-Sets erfolgt derart, dass nach Einführen eines Führungsrohrs von der Körperoberfläche eines Patienten bis zur Oberfläche eines Wirbelkörpers oder Knochens über das Führungsrohr ein Führungsstab mit einem exzentrisch ausgebildeten Hohlraum in den Körper des Patienten bis zur Oberfläche des Wirbelkörpers oder Knochens eingeführt wird, dass die Lage des distalen Endes der Führungskanüle relativ zu einer vorgesehenen Lage, in der Regel unter Röntgensicht, überprüft wird, dass bei Nichtübereinstimmen des vorgesehenen Operationsgebiets mit der Lage des distalen Endes des Führungsrohrs am Wirbelkörper oder Knochen eine exzentrisch am distalen Ende des Führungsstabs ausgebildete distale Spitze gegen die Oberfläche des Wirbelkörpers oder Knochens gedrückt wird, dass das Führungsrohr innerhalb des Führungsstabs in proximaler Richtung zurückgezogen wird, dass der Führungsstab mit dem in ihm befindlichen Führungsrohr um seine distale Spitze verschwenkt wird, um ein Hohlraum des Führungsstabs über das vorgesehene Operationsgebiet zu verschwenken oder dem Operationsbereich näher zu kommen, und dass anschließend das Führungsrohr innerhalb des Führungsstabs wieder distal zur Oberfläche des Wirbelkörpers oder Knochens hin bewegt wird.

Das erfindungsgemäße Instrumenten-Set lässt sich grundsätzlich sowohl transforaminal als auch interlaminar einsetzen.

Durch das erfindungsgemäße Instrumenten-Set mit, wie vorstehend dargelegt, ausgebildetem Führungsstab ist es grundsätzlich möglich, diesen mit der exzentrischen Spitze der distalen Zunge fest gegen die Oberfläche eines Wirbels oder Knochens anzudrücken und dann um die Spitze der Zunge zu verschwenken, wodurch der Hohlraum des Führungsstabs und ein in diesem einsitzendes Führungsrohr mit veschwenkt werden. So kann der Ort des Lumens und des Führungsrohrs von einer nicht passenden Stelle zu einer konkreten Operationsart verschwenkt werden. Gegebenenfalls kann dieser Vorgang mehrmals wiederholt werden.

Die Arbeitsweise mit dem Instrumenten-Set ist daher grundsätzlich derart, dass ein Führungsstab mit einem exzentrisch in seiner Außenkontur und/oder der Symmetrieachse seiner Außenkontur angeordneten Hohlraum in den Körper des Patienten eingeführt wird und/oder dass zunächst ein oder mehrere Dilatoren über Führungsrohr oder Führungsstab bis zum Wirbelkörper eingeführt werden und anschließend die Arbeitshülse über den eingeführten Dilator mit dem größten Durchmesser eingeführt wird und/oder dass zuerst ggf. nach Vornahme eines Hauteinschnitts, eine Hohlnadel mit eingeführtem Stilett in den Körper des Patienten bis zum Wirbelkörper eingestochen wird, anschließend das Stilett entfernt und ein Führungsdraht durch die Hohlnadel eingeführt wird, anschließend nach entfernen der Hohlnadel das Führungsrohr über den Führungsdraht bis zum Wirbelkörper eingeführt wird und schließlich die weiteren Schritte des Verfahrens durchgeführt werden.

Beim Arbeitsablauf kann weiterhin vorgesehen sein, dass zumindest ein Arbeitsinstrument durch die Arbeitshülse hindurch bis zum Operationsort am Wirbelkörper oder Knochen eingeführt und die vorzunehmende Operation, wie das Entfernen von Osteophyten, an der Wirbel- oder Knochenoberfläche ausgeführt wird oder dass der Schaft eines Endoskops durch einen länglichen Hohlraum des Arbeitsinstruments eingeführt wird.

Weiterhin kann vorgesehen sein, dass zunächst ein Endoskop durch die Arbeitshülse eingeführt wird und anschließend durch einen länglichen Hohlraum des Endoskops ein Arbeitsinstrument bis zur Oberfläche des Wirbelkörpers oder Knochens eingeführt wird und ein Operationsschritt, wie das Entfernen eines Osteophyten, unter Endoskopsicht ausgeführt wird.

Durch die Erfindung wird insgesamt die Möglichkeit geschaffen, bei nicht punktgenau auf eine Operationsstelle, wie auf einem Wirbelkörper oder einem Knochen, eingeführtem Instrument bzw. Instrumenten-Set eine Repositionierung durch Verschwenken zu erreichen, so dass der genaue Operationsort zielgenau - unter Röntgen - und/oder Endoskopsicht von den distalen Enden der vorzusehenden Instrumente eingenommen werden kann.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt:
- Fig. 1: einen Teil-Instrumentensatz zum Einführen und Positionieren einer Arbeitshülse;
- Fig. 1a: einen aufsetzbaren Griff eines Führungsstabs des Teil-Instrumentensatzes der Fig. 1;
- Fig. 2: eine Seitenansicht auf einen erfindungsgemäβen Führungsstab;
- Fig. 2a: eine Seitenansicht versetzt um einen Winkel von 90° um die Achse des Führungsstabs auf diesen;
- Fig. 2b: einen Längsschnitt durch den Führungsstab der Fig. 1a, 1b;
- Fig. 2c: eine Sicht auf das distale Ende des Führungsstabs;
- Fig. 2d: eine Sicht auf das proximale Ende des Führungsstabs;
- Fig. 3: eine Arbeitshülse des erfindungsgemäßen Instrumenten-Sets;
- Fig. 3a: eine vergrößerte Darstellung des distalen Endes der Arbeitshülse;
- Fig. 4: einen Arbeitsinstrumenten-Satz mit
- Fig. 4a: einem Fräser mit
- Fig. 4b: einem aufsetzbaren Griff für den Fräser mit
- Fig. 4c: einem Meißel;
- Fig. 5: eine Führungshülse;
- Fig. 6: ein endoskopisches Teil eines erfindungsgemäßen Instrumenten-Satzes;
- Fig. 7: Darstellungen zum Verfahrensablauf des Einsatzes des Instrumenten-Satzes der Fig. 1 mit
- Fig. 7a: der Darstellung eines ersten Verfahrensschrittes mit Einführen des distalen Endes einer mit einem Obturator verschlossenen Hohlnadel bis in das Operationsgebiet an einem Wirbel;
- Fig. 7b: den weiteren Verfahrensschritt eines Einführens eines Führungsdrahts in die Hohlnadel nach Entfernen des Obturators;
- Fig. 7c: der Schritt des Einführens des Führungsrohrs über den Führungsdraht;
- Fig. 7d: Einführung des exzentrischen Führungsstabs mit Handgriff in einer ersten Winkelstellung über das Führungsrohr;

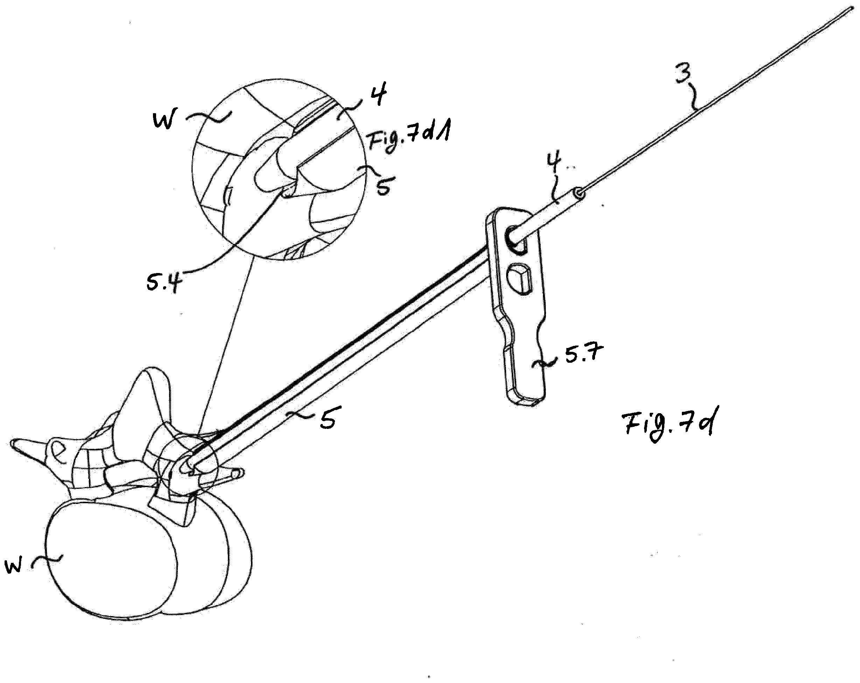

- Fig. 7d1: eine vergrößerte Darstellung des distalen Bereichs der Teile der Fig. 7d;

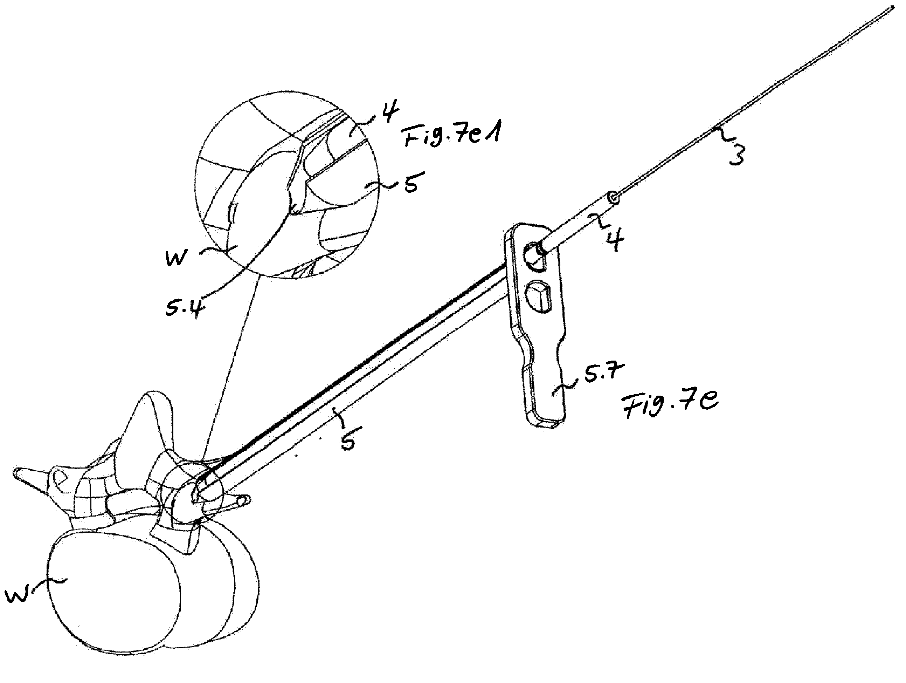

- Fig. 7e: Zurückziehen des Führungsrohrs in proximaler Richtung innerhalb des exzentrischen Führungsstabs bei beibehaltener Winkelstellung, so dass der exzentrische Führungsstab mit seiner Spitze auf dem Wirbel aufsitzt;
- Fig. 7e1: eine vergrößerte Darstellung des distalen Bereichs der Teile der Fig. 7e;

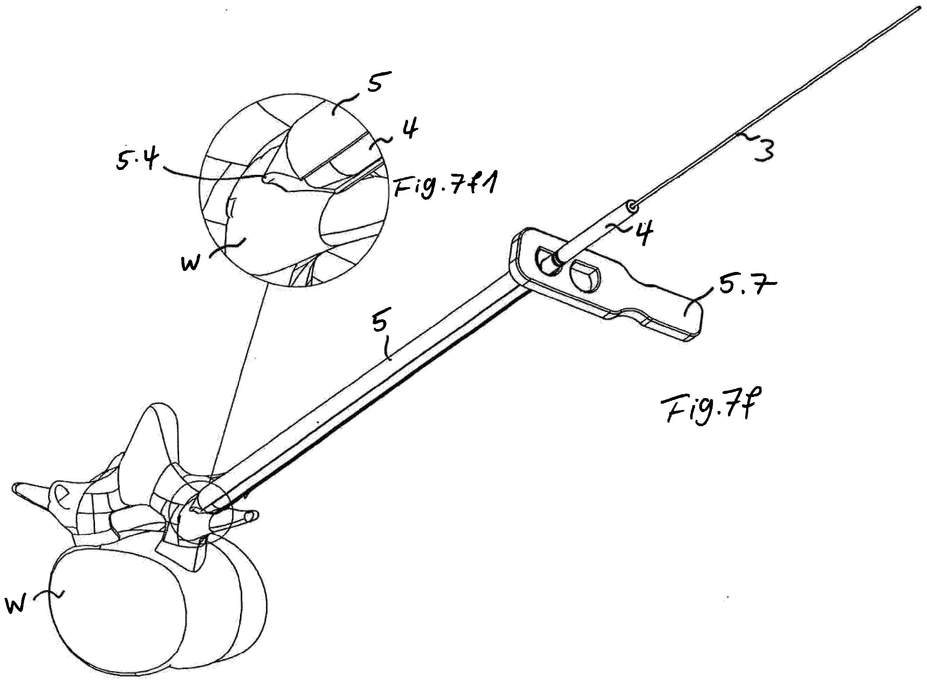

- Fig. 7f: exzentrischer Führungsstab mit eingeschobenem Führungsrohr in einer um 90° um die distale Spitze des Führungsstabs relativ zur Ausrichtung der Fig. 7d bis 7e1 verschweißten Stellung des exzentrischen Führungsstabs;
- Fig. 7f1: eine vergrößerte Darstellung des distalen Bereichs der Fig. 7f;

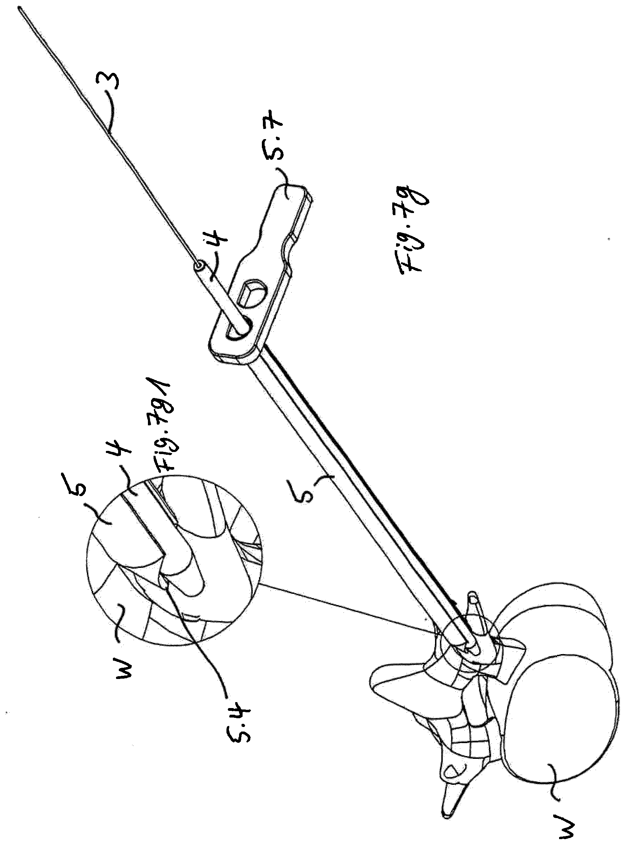

- Fig. 7g: Vorschieben des Führungsrohrs bis zur Anzeige am Wirbel und Zurückziehen des exzentrischen Führungsrohrs in der Winkelposition der Fig. 7f;
- Fig. 7g1: eine vergrößerte Darstellung des distalen Bereichs der Teile der Fig. 7g;

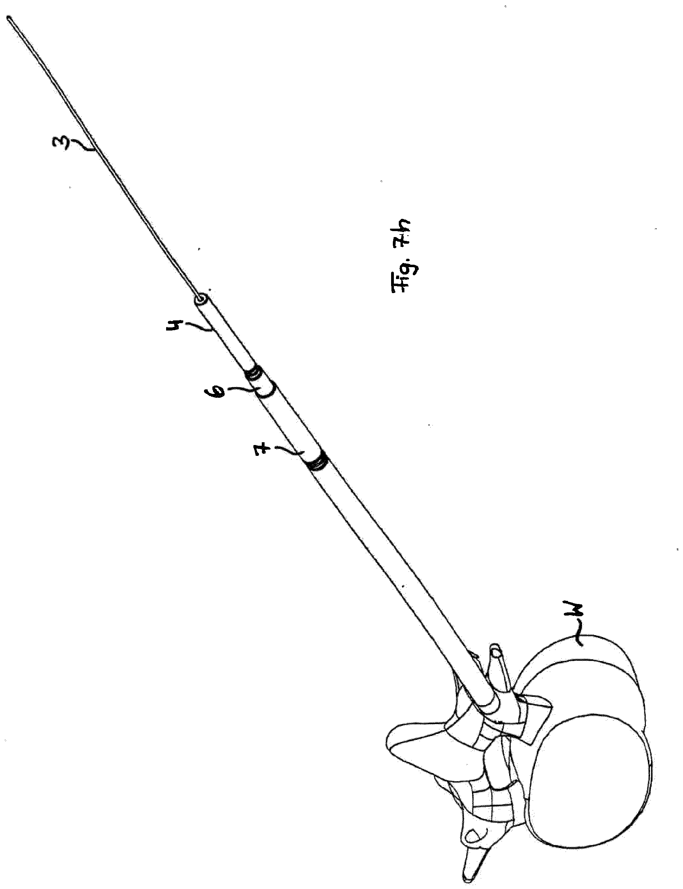

- Fig. 7h: eine Darstellung nach Entfernen des exzentrischem Führungsstab und nach Entfernen zweier Dilatoren über das Führungsrohr;

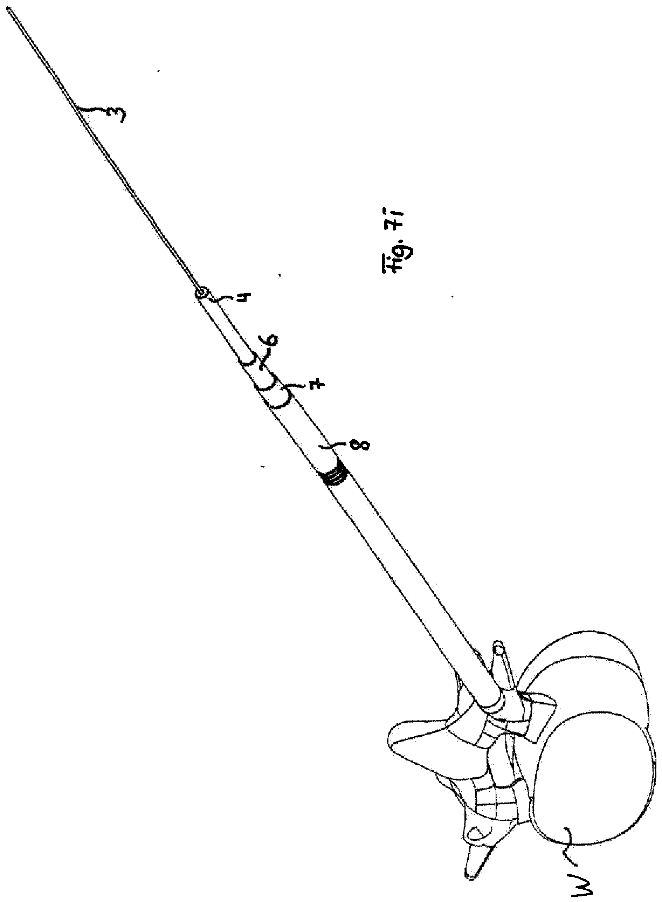

- Fig. 7i: eine Darstellung nach Einführen eines dritten Dilators;

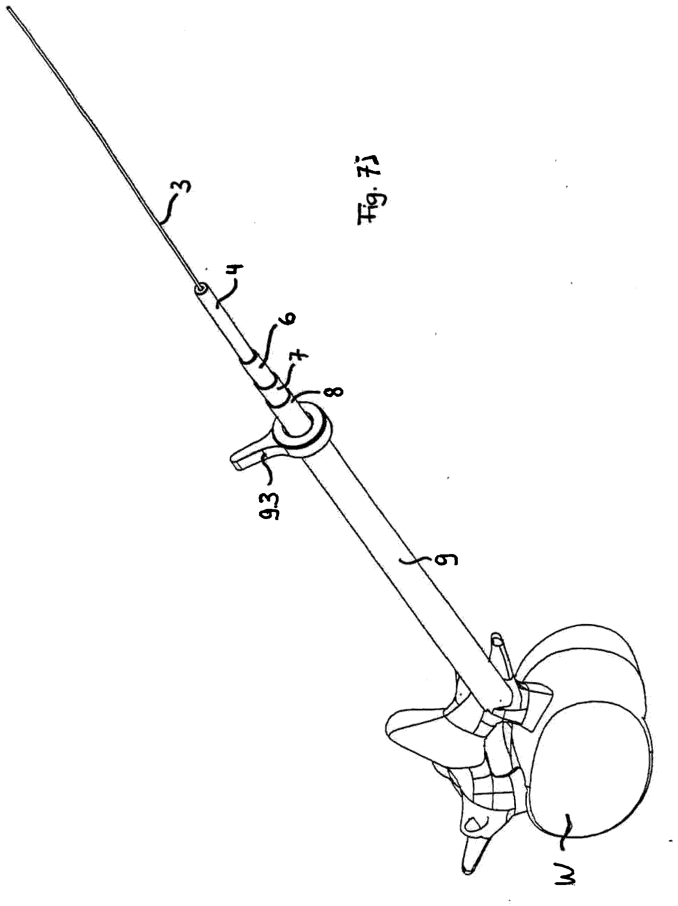

- Fig. 7j: über den zweiten Dilator eingeführte Arbeitshülse mit Handgriff;

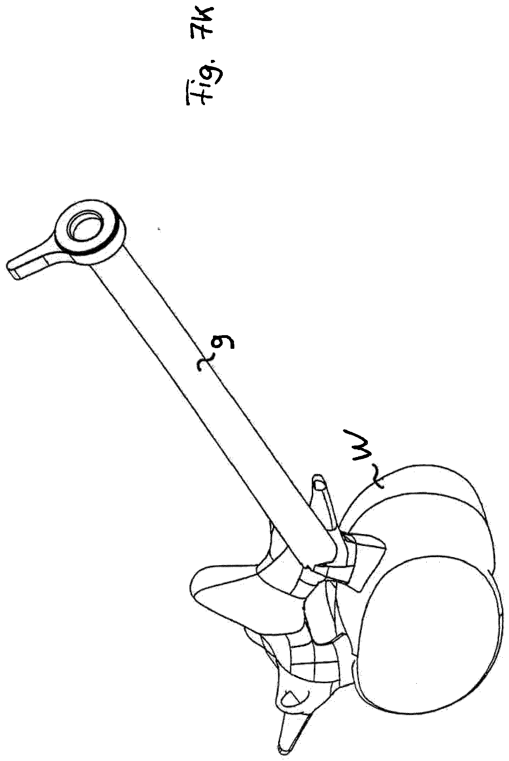

- Fig. 7k: nach Entfernen der anderen Elemente lediglich am Wirbel anliegende Arbeitshülse mit Handgriff;

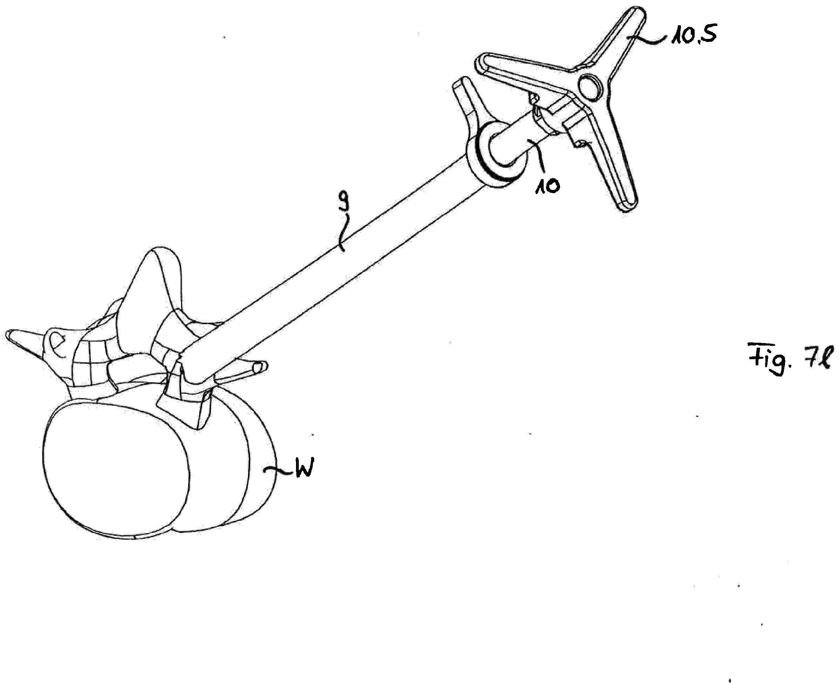

- Fig. 7l: Einführen eines Fräsers mit Flügelgriff in die Arbeitshülse;

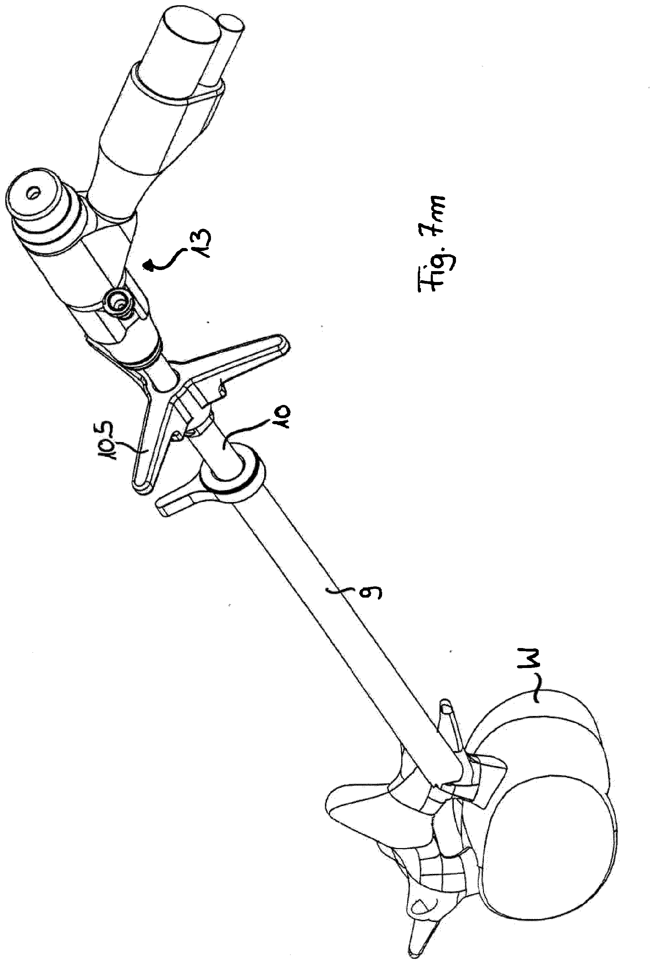

- Fig. 7m: durch den mit einem Hohlrohr versehenen Fräser eingeführtes Endoskop;

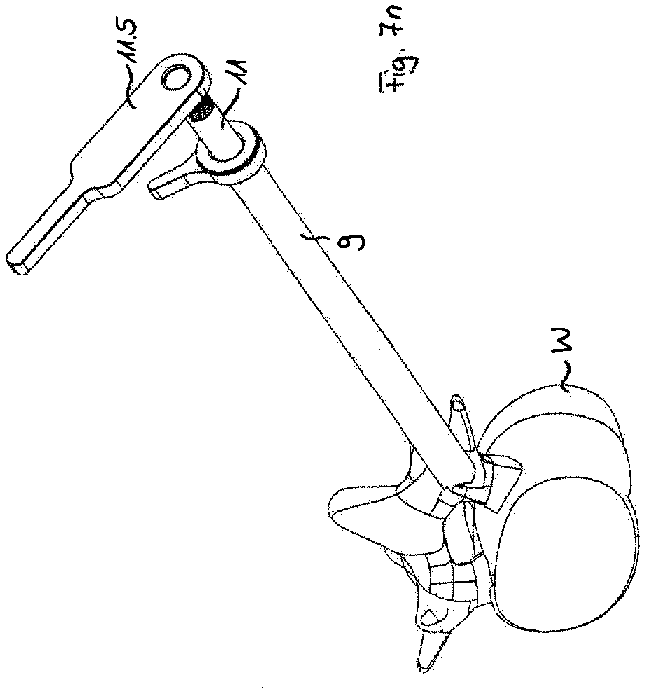

- Fig. 7n: nach Entfernen von Fräser und Endoskop durch die Arbeitshülse eingeführter mit einem Handgriff versehener Meißel;

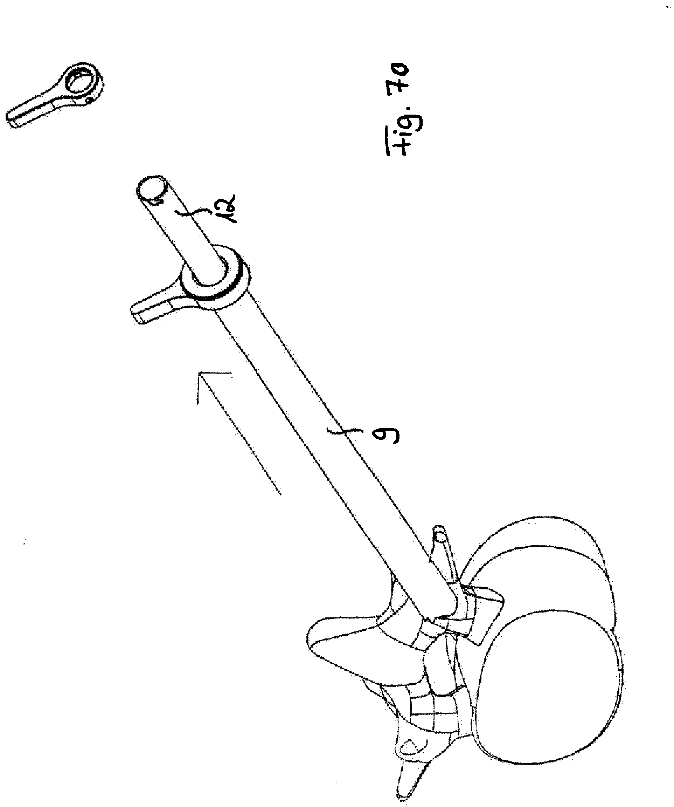

- Fig. 7o: Einsetzen einer Führungshülse in die Arbeitshülse und anschließendes Entfernen letzterer; und

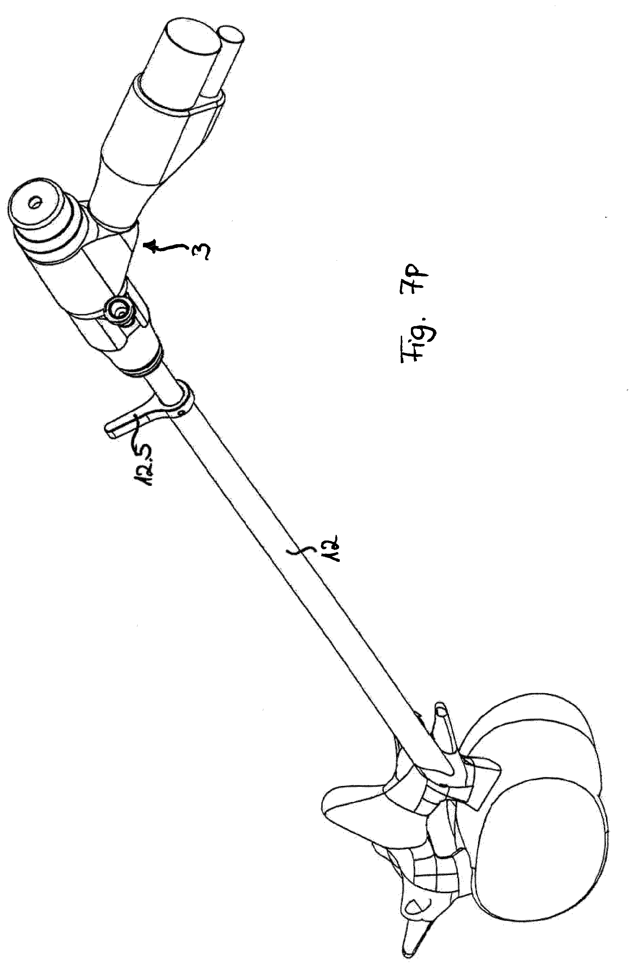

- Fig. 7p: Einführen eines Endoskops in die Führungshülse.

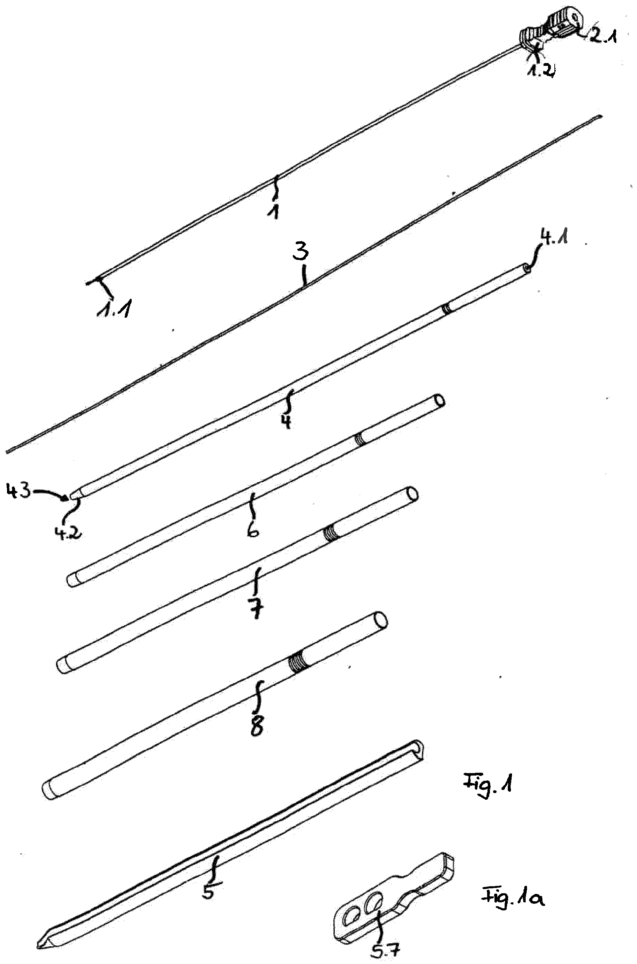

Wie in der Fig. 1 dargestellt, weist ein erfindungsgemäßes Instrumentenset im Wesentlichen die folgenden Teile auf:
Eine Hohlnadel 1 mit abgeschrägtem distalem Ende 1.1 und einem Griffteil 1.2. In die Hohlnadel 1 ist ein Stilett (selbst nicht sichtbar) mit einem Handgriff 2.1 eingeschoben, dessen distale Stirnseite mit dem abgeschrägten distalen Ende 1.1 der Hohlnadel fluchtet. Weiterhin weist das Instrumentenset einen Führungsdraht 3 auf, dessen Durchmesser an das Lumen des zylindrischen Hohlraums der Hohlnadel 1 angepasst ist. Ein anderes Teil des Sets ist ein hohles zylindrisches Führungsrohr 4, dessen Lumen vorzugsweise ebenfalls an den Durchmesser des Führungsdrahts angepasst ist.

Ein weiteres wesentliches Teil des Instrumentensets ist ein exzentrischer Führungsstab 5, dessen Hohlraum (Lumen) der Außenkontur des Führungsrohrs 4 angepasst ist. Zum exzentrischen Führungsstab 5 gehört ein auf das proximale Ende desselben drehfest aufsetzbarer Griff 5.7 (Fig. 1a). Weitere Teile sind ein erster Dilator 6, ein zweiter und ein dritter Dilator 7, 8, wobei die Hohlräume des ersten Dilators 6 ebenfalls an die Außenkontur des Führungsstabs 5 und die Hohlräume des zweiten Dilators 7 und des dritten Dilators 8 an die Außenkontur des ersten Dilators 6 bzw. des zweiten Dilators 7 angepasst sind.

Die vorgenannten Teile 4 bis 8 sind an ihrem distalen Ende außenseitig konisch verjüngt, so dass sich ein kontinuierlicher Übergang von einem innen liegenden Teil zu einem au-βen liegenden Teil ergibt.

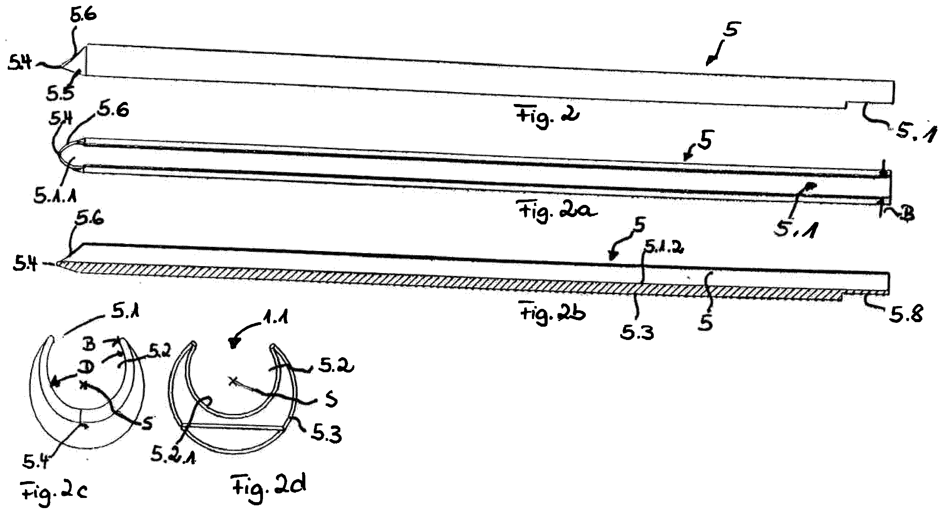

Die Fig. 2a bis 2d zeigen einen erfindungsgemäßen exzentrischen Führungsstab 5 mit einer mit einer seitlichen Schlitzöffnung 5.1 versehenen teil-zylindrischen Nut 5.2 als Hohlraum. Die Außenwandfläche oder Außenkontur 5.3 des Führungsstabs 5 ist - bis auf die Schlitzöffnung 5.1 - über den größten Teil der Länge teilzylindrisch ausgebildet mit einer Symmetrieachse S der zylindrischen Außenkontur des Führungsstabs (Fig. 2c, 2d). Die Nut 5.2 ist exzentrisch zu der Symmetrieachse S ausgebildet. An seinem distalen Ende geht die Schlitzöffnung 5.1 in einen distalen Öffnungsbereich 5.1.1 über, so dass das distale Ende des Führungsstabs 5 durch eine distale Spitze 5.4 gebildet wird, die ebenfalls exzentrisch zur Symmetrieachse S liegt. Sowohl die Außenwandfläche 5.3 als auch die die Nut 5.2 umgebende Innenwandfläche 5.2.1 des Führungsstabs 5 sind - bis auf die seitliche Schlitzöffnung 5.1 teilzylinderförmig ausgebildet.

Kanten 5.6 im distalen Endbereich des Führungsstabs 5 verlaufen in der seitlichen Draufsicht der Fig. 2a konvex bogenförmig in die distale Spitze 5.4 einer distalen Zunge 5.5, wobei sich im Schnitt der Fig. 2b eine leicht konkave Form der Kanten 5.6 zeigt.

Am proximalen Ende weist der Führungsstab 5 eine seitliche Abflachung 5.8 der Längswandung auf, so dass ein Griff 5.7 (beispielsweise Fig. 1a) drehfest aber lösbar mit dem proximalen Ende des Führungsstabs 5 verbunden werden kann.

Die Ausgestaltung des exzentrischen Führungsstabs 5 mit der sowohl exzentrisch zur Symmetrieachse S (Fig. 2c, 2d) ausgebildeten Nut 5.2 als auch der exzentrisch zur Symmetrieachse S ausgerichteten Spitze 5.4 dient dazu, durch Verschwenken des Führungsstabs 5 um die an der Wandung eines Wirbelkörpers 6 der Wirbelsäule - im Folgenden kurz Wirbel W - aufsitzende Spitze 5.4 den distalen Öffnungsbereich der Nut 5.2, deren Position den Auftreffort von Instrumenten an der Außenwandung eines Wirbels W bestimmt, zu verlagern, um so bei nicht zielgenauem Einführen der zunächst eingeführten Instrumente eines Instrumenten-Sets, von dem der Führungsstab 5 ein Teil ist, eine genaue Positionierung der Nutz 5.2 und damit des durch diese bestimmten Eintritts- und Austrittsorts von Instrumenten an der Außenwand eines Wirbels zu korrigieren, um so beispielsweise dort störende Osteophyten durch entsprechende Werkzeuge entfernen zu können.

Bei dem einen zylindrischen Hohlraum 4.1 aufweisenden Führungsrohr 4 (Fig. 1) handelt es sich im Wesentlichen um ein Führungsrohr, wie es üblicherweise als Teil eines Instrumenten-Satzes oder -Sets für Wirbeloperationen eingesetzt wird. Das Führungsrohr 4 ist über den größten Teil seiner Länge zylindrisch ausgebildet und weist einen zylindrischen Hohlraum auf. Der distale Endbereich 4.2 verläuft leicht konisch nach innen und kann ein abgeschrägtes Ende mit einer ebenfalls schräg zu seiner Längsachse verlaufenden distalen Öffnung 4.3 aufweisen.

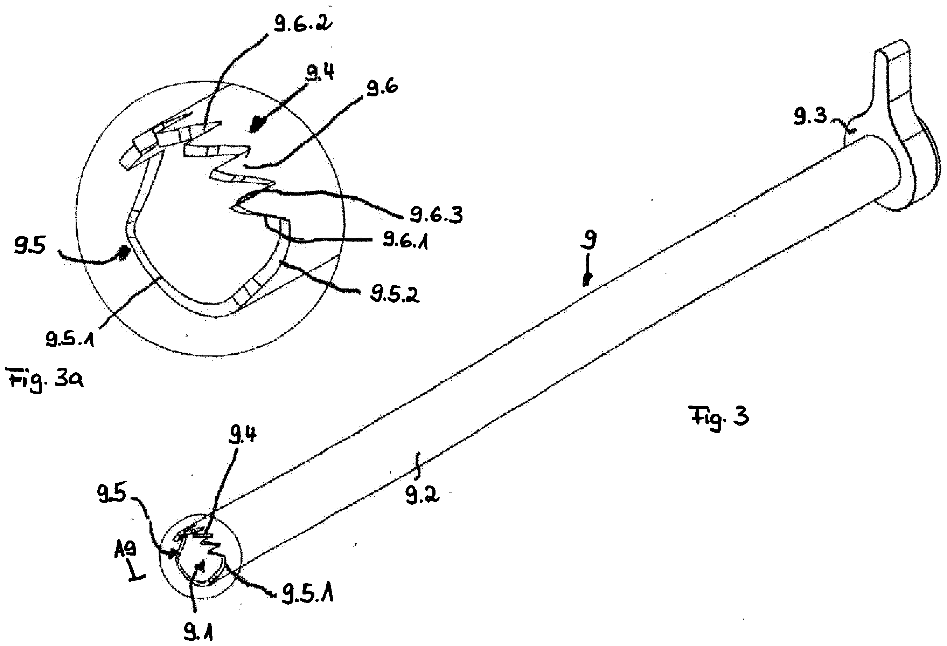

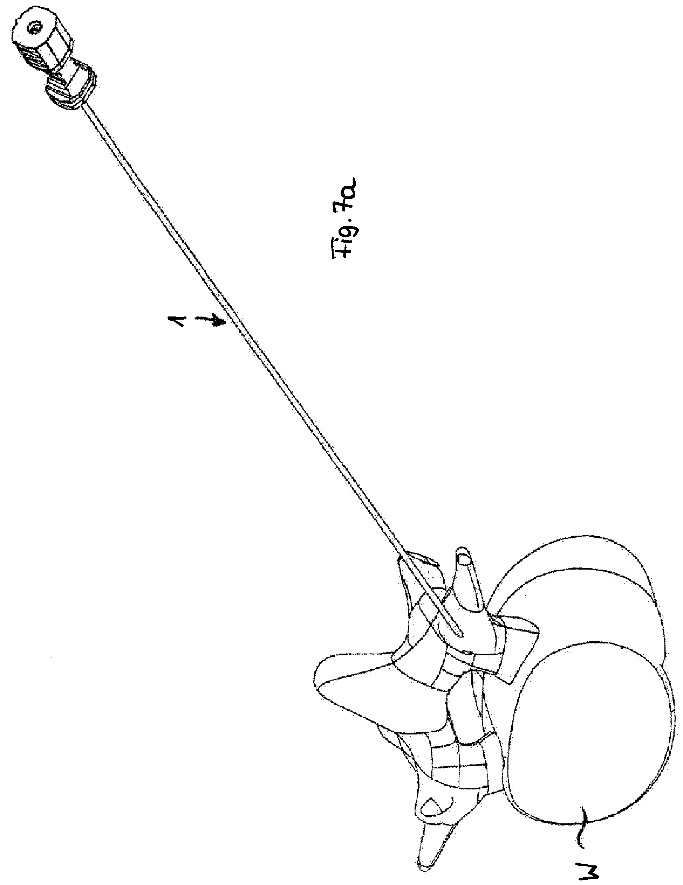

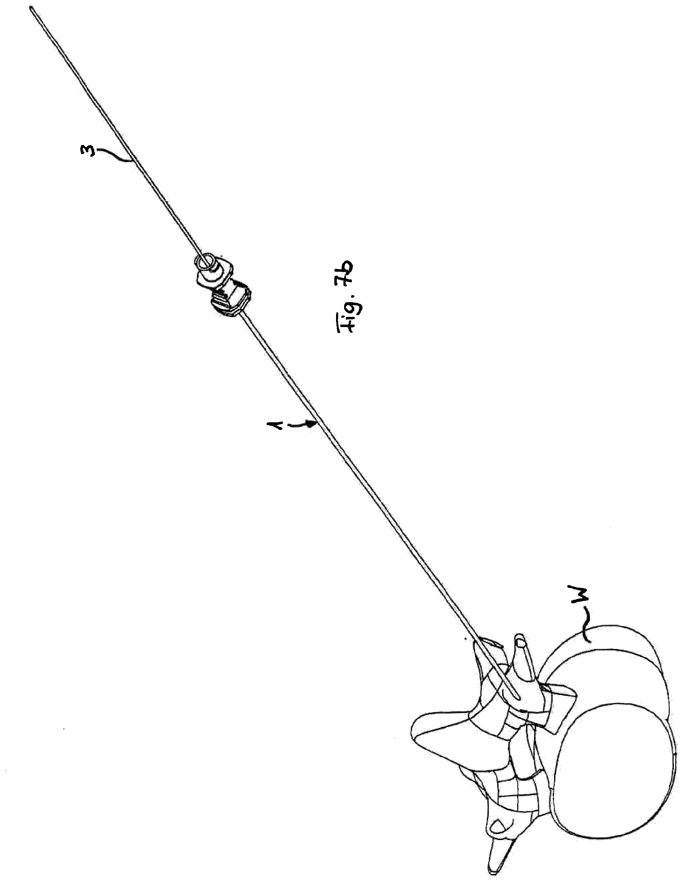

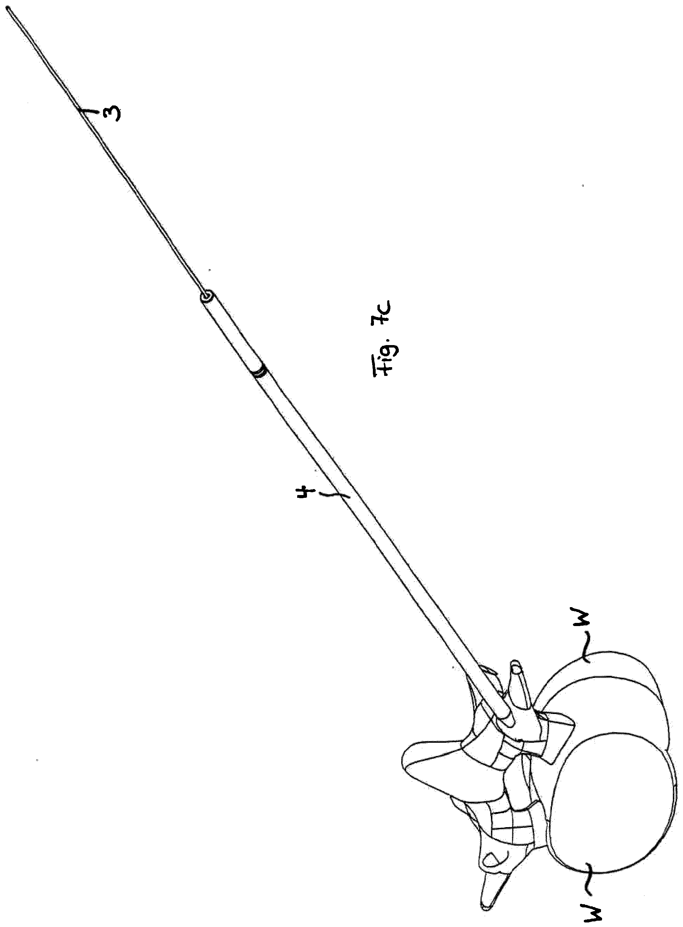

Die Fig. 3 zeigt eine Arbeitshülse 9, die über das Führungsrohr und gegebenenfalls den exzentrischen Führungsstab 5, gegebenenfalls unter vorherigem Einsatz von über den Führungsstab 5 geschobenen Dilatoren 6,7,8 mit seinem distalen Ende bis zum Arbeits-/Operationsort eingeschoben wird und durch welches - nach Entfernen von Führungsrohr 4 und gegebenenfalls Führungsstab 5 sowie Dilatoren 6.8 - Arbeitsinstrumente direkt oder zunächst ein Endoskop und durch einen zylindrischen Hohlraum des Endoskops, dann Arbeitsinstrumente, wie Fräser, Meißel, Zangen oder dergleichen eingeführt werden (zum Arbeitsablauf auch Fig. 7).

Die Arbeitshülse 9 weist einen einen ebenfalls zylindrischen Hohlraum 9.1 umgebenden zylindrischen Mantel 9.2 auf. An seinem proximalen Ende ist sie mit einem Griffteil 9.3 versehen. Das distale Ende weist eine sich über einen Teil des Umfangs des Mantels 9.2 erstreckende Zahnreihe 9.4 sowie dieser gegenüberliegend eine Lippe 9.5 auf.

Zähne 9.6 der Zahnreihe 9.4 sind asymmetrisch ausgebildet. Eine erste Flanke 9.6.1 verläuft im Wesentlichen achsparallel zur Mittelachse A9 der Arbeitshülse 9, während die gegenüberliegende zweite Flanke 9.6.2 als relativ zur Erstreckung der Achse A9 geneigtes flaches S ausgebildet ist. Die distale Stirnseite 9.5.1 der Lippe 9.5 liegt auf der gleichen axialen Höhe wie die distalen Spitzen 9.6.3 der Zähne 9.6 und sie geht beidseitig über Bögen 9.5.2 in den Zahngrund der jeweils äußeren Zähne 9.6 der Zahnreihe 9.4 über.

Mittels der Zähne 9.6 kann die Arbeitshülse 9 am Knochenmaterial eines Wirbels oder Knochens sicher verankert werden, während die distale Lippe 9.5 dazu dient empfindliche Körperelemente, wie Nerven, zu schützen, insbesondere gegenüber durch die Arbeitshülse 9 direkt oder indirekt eingeführte Werkzeuge zum Arbeiten an der Oberfläche des Wirbels W oder Knochens.

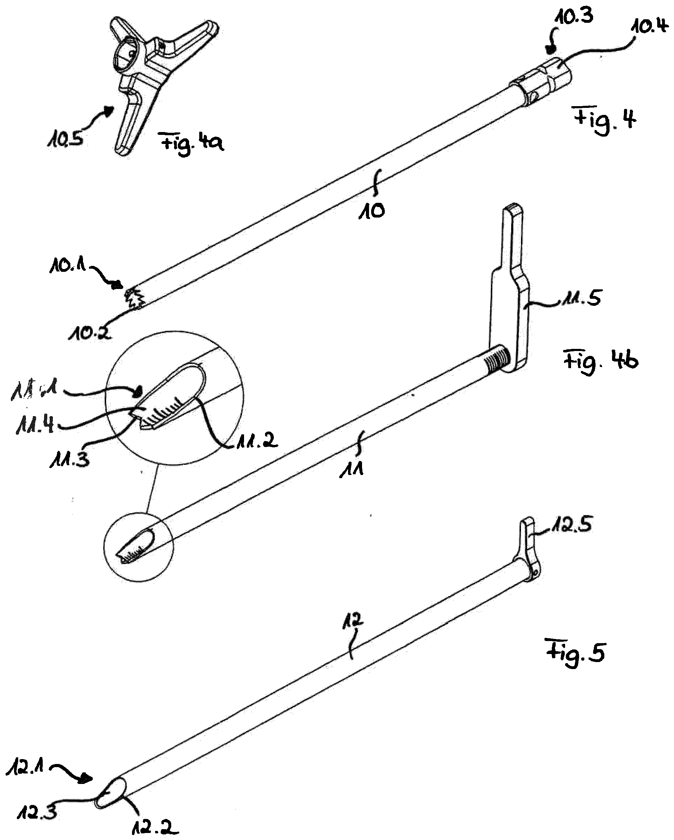

Die Fig. 4 zeigt ein als Fräser 10 bezeichnetes Arbeitswerkzeug zum Entfernen von Knochenmaterial an Knochen, wie Osteophyten. Der Fräser 10 weist ein distales Ende 10.1 mit einer über den gesamten Umfang umlaufenden stirnseitigen Zahnreihe 10.2 auf, deren Zähne derart asymmetrisch ausgebildet sind, dass eine Flanke eines Zahns - die Vorderflanke - parallel zur Achse A10 des Fräsers 10 verläuft, während die andere Flanke einen Winkel einschließt. Das proximale Ende 10.3 des Fräsers 10 ist nicht kreissymmetrisch ausgebildet, sondern mit einer oder mehreren Abflachungen 10.4 am Umfang versehen, so dass hier drehfest ein Sterngriff 10.5 (Fig. 4a) aufgesetzt werden kann, mit denen der Fräser 10 zum Entfernen von Knochenmaterial mittels seiner distalen Zahnreihe 10.2 gedreht werden kann.

Ein weiteres Arbeitswerkzeug ist ein Meißel 11, wie er in der Fig. 4b dargestellt ist, dieser weist ein distales Ende 11.1 mit einer Abschrägung 11.2 auf. Die distale Stirnseite 11.3 der durch die Abschrägung 11.2 gebildeten Zunge 11.4 ist ebenfalls gezähnt oder mit wenigen Zähnen (hier drei Zähne) ausgebildet. Auch hierdurch kann Knochenmaterial entfernt werden. Zur (Dreh-)Bewegung des Meißels 11 ist dieser an seinem proximalen Ende mit einem Griff 11.5 drehfest versehen, der gegebenenfalls ebenfalls zerstörungsfrei entfernt werden kann. Der Außendurchmesser der Teile 10, 11 ist vorzugsweise an das Lumen der Arbeitshülse 9 angepasst. Fig. 5 zeigt eine Führungshülse 12, die ebenfalls am distalen Ende 12.1 eine Abschrägung 12.2 aufweist, durch die eine seitliche Zunge 12.3 gebildet wird, die aber im Gegensatz zum Meißel 11 nicht als Werkzeug ausgebildet ist. Am proximalen Ende der Führungshülse 12 ist ebenfalls ein Griff 12.5 drehfest angebracht, der gegebenenfalls auch zerstörungsfrei entfernbar ist. Der Außendurchmesser der Führungshülse 12 entspricht dem Lumen der ersten Arbeitshülse 9.

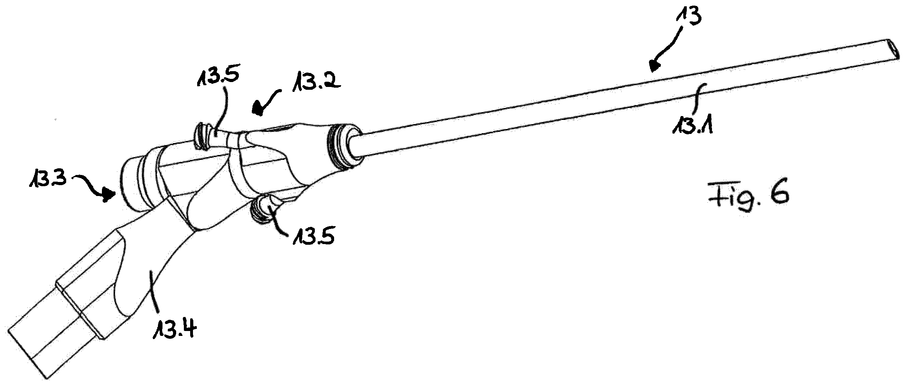

Die Fig. 6 zeigt schematisch ein Endoskop 13 mit einem Rohrteil 13.1 und einem Kopfteil 13.2. Dieses weist eine axiale Eingangsöffnung zum Einführen von Kreuzinstrumenten auf. Weiterhin weist es einen abgewinkelten Ansatz zum Anschluss 13.4 eines Bildwiedergabegeräts (Monitor) sowie Spülanschlüsse 13.5 auf. Das Endoskop ist im Inneren, insbesondere des zylindrischen Rohrteils, in üblicher Weise ausgebildet, insbesondere mit einem Arbeitskanal mit relativ großem Durchmesser im Verhältnis zum Außendurchmesser des Rohrteils sowie einseitig an diesem angeordnetem Lichtleiter bzw. Kamera und Lichtquelle sowie ein oder zwei Spülkanälen.

Der Verfahrensablauf des Einsatzes des erfindungsgemäßen Werkzeug-Satzes, zu deren Teile das Führungsrohr 4 des Führungsstabs 5, sowie die in Bezug auf die Fig. 1 beschriebenen weiteren Teile gehören, erfolgt im Wesentlichen folgendermaßen (Fig. 7):
Zunächst erfolgt ein Einschnitt in der Haut des Patienten nahe dem zu bearbeitenden Wirbel W. Durch diesen Einschnitt wird dann die distal abgeschrägte Hohlnadel 1 zusammen mit dem in diesem befindlichen Stilett unter Röntgensicht bis zum Operationsort am Wirbel W eingeführt (Fig. 7a). Anschließend wird das Stilett entfernt und der Führungsdraht 3 durch die Hohlnadel ebenfalls bis zu deren distalem Ende geführt (Fig. 7b). Im Folgenden wird die Hohlnadel 1 entfernt und dann über den liegenden Führungsdraht 3 der Führungsrohr 4 bis zum Operationsgebiet am Wirbel W eingeführt (Fig. 7c). Im Folgenden könnte über den Führungsrohr 4 ein erster Dilator 6 eingeschoben werden, was aber im Folgenden nicht der Fall ist.

Insofern wird beispielhaft auf die WO 2014/146797 A1 oder die WO 2015/022040 A1 verwiesen. Nach Aufsetzen des Führungsrohrs 4 oder auch des Dilators 6 auf der Außenwandung eines Knochens oder Wirbels W in einem zu bearbeitenden/zu operierenden Bereich, wie beschrieben, ist der weitere Verfahrensablauf der folgende, wie er ab Fig. 7d dargestellt ist:
Über das Führungsrohr 4 wird der exzentrische Führungsstab 5 bis zur Oberfläche des Knochens oder Wirbels W in distaler Richtung eingeführt (Fig. 7d, Fig. 7d1), sodann wird die Positionierung des Führungsrohrs 4 (und gegebenenfalls auch des Dilators 6) relativ zum gewünschten Operationsort, beispielsweise dem Ort von Osteophyten unter Röntgensicht geprüft und, wenn die erste Positionierung des Führungsrohrs 4 nicht derart ist, dass in nachfolgenden Schritten eine über diese und/oder den Führungsdraht einzubringende Arbeitshülse einen entsprechenden Osteophyt überdeckt oder erfasst, so dass er dann durch einzuführende Arbeitsinstrumente bearbeitet werden kann, wird das Führungsrohr 4 (gegebenenfalls mit Dilator) abgehoben von der Oberfläche des Wirbels W oder Knochens, während der exzentrische Führungsstab 5 mit seiner distalen Spitze 5.4 auf dem Wirbel aufsitzt (Fig. 7e, Fig. 7e1).

Im Folgenden wird dann der exzentrische Führungsstab 5 um seine auf der Wandung des Wirbels W oder Knochens aufsitzende Spitze 5.4 in der gewünschten Weise mittels des Hebels oder Griffs 5.7 verschwenkt, so dass sein Hohlraum und damit das in diesem einsitzende Führungsrohr 4 über den gewünschten Operationsbereich, beispielsweise die Position eines Osteophyten an Wirbel W oder Knochen gelangt - Übergang von der Fig. 7e zur Fig. 7f, Fig. 7f1.

In einem weiteren Schritt wird dann das Führungsrohr 4 wieder in distaler Richtung bis zum Anliegen an der Wandung des Wirbels/Knochens W innerhalb des Führungsstabs 5 nach vorne geschoben, wobei die Winkelausrichtung des Führungstabs 5 und sein Griff 5.7 beibehalten wird. Anschließend wird der exzentrische Führungsstab 5 in proximaler Richtung zurückgezogen, so dass dessen distales Ende sich von der Wandung des Wirbels/Knochens W löst (Übergang von Fig. 7f zu Fig. 7g, Fig. 7g1) und anschließend mittig entfernt. Zum Aufweiten des von der Körperoberfläche zum Wirbel/Knochen führenden Arbeitskanals werden gegebenenfalls Dilatoren 6, 7, 8 in den Körper des Patienten bis zum Arbeitsort am zu behandelnden Wirbel W oder Knochen eingeführt, wenn ein größerer Querschnitt des Kanals gewünscht ist (Fig. 7h, 7i).

Nach Einführen der Arbeitshülse 9 über die Teile 4 und gegebenenfalls 6, 7, 8 (Fig. 7j) werden sämtliche anderen Rohre, wie das Führungsrohr 4 und gegebenenfalls die Dilatoren 6, 7, 8 entfernt (Fig. 7k). Anschließend werden gegebenenfalls Arbeitswerkzeuge, wie Fräser 10 der Fig. 4 oder der Meißel 11 der Fig. 4c, Zangen zur Durchführung der Operationsschritte am Wirbelkörper/Knochen unmittelbar durch die Arbeitshülse 9 bis zum Operationsort eingeführt (Fig. 7l bis 7n). Um mit dem Fräser 10 mit einem Hohlraum unter Sicht zu arbeiten, kann durch diesen ein Endoskop mit seinem distalen Ende bis nahe dem Operationsort eingeführt werden.

Nach Entfernen der distal mit Zähnen 9.6 versehenen Arbeitshülse 9 (Schritt Fig. 7o) kann gegebenenfalls über den Meißel 11 und dessen entfernbaren Griffs 11.5 eine - wie unter Bezug auf Fig. 5 dargelegt - anders ausgestaltete Führungshülse 12 eingeführt werden und durch diese nach Entfernen des Meißels 11 wiederum ein Endoskop 13 (Fig. 7p) durch den Kanal gemäß weiterer Arbeitswerkzeuge zum weiteren Bearbeiten an der Operationsnut eingeführt werden.

Durch den erfindungsgemäßen exzentrisch ausgebildeten Führungsstab 5 ist es daher möglich bei nicht zielgenauem Einführen der Arbeitsinstrumente in den ersten Verfahrensschritten einen seitlichen Versatz des Operationsortes am Wirbel/Knochen durch Verschwenken des Führungsstabs um dessen an der Oberfläche des Wirbels/Knochens festsitzenden Spitze zu erreichen.

Durch die entsprechende Arbeitshülse 9 können Arbeitsinstrumente 10, 11, gegebenenfalls auch ein Endoskop 13 mit einem Kanal für Arbeitsinstrumente eingeführt werden, um dort - gegebenenfalls unter endoskopischer Sicht - die entsprechenden Operationsschritte auszuführen.

## Patentansprüche

1. Instrumenten-Set für Wirbelsäulenoperationen mit mindestens einem Dilator (6,7,8), mit einem Führungsrohr (4) und mit einem Führungsstab (5) mit einem exzentrischen Hohlraum, wobei das Führungsrohr (4) im Hohlraum des Führungsstabs (5) aufnehmbar ist, wobei der Führungsstab (5) eine seitliche mit dem Hohlraum verbundene Schlitzöffnung (5.1) aufweist, so dass der Hohlraum eine teilzylindrische Nut (5.2) bildet, und die Breite (B) der Schlitzöffnung (5.1) kleiner ist als der Durchmesser (D) der Nut (5.2), und wobei der Führungsstab (5) eine distale Zunge (5.5) aufweist, die exzentrisch zum Hohlraum des Führungsstabs (5) angeordnet ist.

2. Set nach Anspruch 1, **dadurch gekennzeichnet, dass** der Hohlraum exzentrisch zur Außenkontur (5.3) und/oder zur Symmetrieachse (S) der Außenkontur (5.3) des Führungsstabs (5) ausgebildet ist.

3. Set nach Anspruch 1 oder 2, **dadurch gekennzeichnet, dass** die distale Zunge (5.5) des Führungsstabs (5) exzentrisch zu einer Symmetrieachse (S) des Führungsstabs (5) angeordnet ist.

4. Set nach einem der vorangehenden Ansprüche, **dadurch gekennzeichnet, dass** die den Hohlraum bildende Nut umgebende Innenwandfläche (5.2.1) des Führungsstabs (5) teilzylindermantelförmig ist.

5. Set nach einem der vorangehenden Ansprüche, **dadurch gekennzeichnet, dass** die Außenwandfläche (5.3) des Führungsstabs (5) teilzylindermantelförmig ist.

6. Set nach einem der vorangehenden Ansprüche, **dadurch gekennzeichnet, dass** die Schlitzöffnung (5.1) der Nut (5.2) des Führungsstabs (5) der distalen Zunge (5.5) diagonal gegenüber liegt.

7. Set nach einem der vorangehenden Ansprüche, **dadurch gekennzeichnet, dass** die distale Zunge (5.5) des Führungsstabs (5) einander gegenüberliegende teilbogenförmige Kanten (5.6) aufweist, deren distale Enden in einer Spitze (5.4.1) der distalen Zunge (5.5) münden.

8. Set nach einem der vorangehenden Ansprüche, **gekennzeichnet durch** eine Arbeitshülse (9) mit einem sich längserstreckenden, insbesondere zylindrischen Hohlraum (9.1), das einen dem Querschnitt des Führungsrohrs (4), vorzugsweise aber dem Querschnitt des Führungsstabs (5) mindestens gleichen Querschnitt aufweist.

9. Set nach Anspruch 8, **dadurch gekennzeichnet, dass** die Arbeitshülse (9) an seiner distalen Stirnseite eine Zahnreihe (9.4) aufweist.

10. Set nach Anspruch 9, **dadurch gekennzeichnet, dass** die Zahnreihe (9.4) sich über einen Teil des Umfangs der Arbeitshülse (9) erstreckt.

11. Set nach Anspruch 10, **dadurch gekennzeichnet, dass** sich die Zahnreihe (9.4) über die Hälfte des Umfangs der Arbeitshülse (9) erstreckt.

12. Set nach einem der Ansprüche 9 bis 11, **dadurch gekennzeichnet, dass** die Zähne (9.6) der Zahnreihe (9.4) asymmetrisch ausgebildet sind.

13. Set nach einem der Ansprüche 9 bis 12, **dadurch gekennzeichnet, dass** eine erste Flanke (9.6.1) der Zähne (9.6) als flaches zur distalen Spitze (9.6.3) eines Zahns (9.6) hin geneigtes S ausgebildet ist.

14. Set nach einem der Ansprüche 9 bis 13, **dadurch gekennzeichnet, dass** eine zweite Flanke (9.6.2) achsparallel ausgerichtet ist.

15. Set nach einem der Ansprüche 8 bis 14, **dadurch gekennzeichnet, dass** sich über einen Teil des Umfangs der Arbeitshülse (9) eine distale Lippe (9.5) erstreckt, wobei insbesondere die Spitzen (9.6.3) der Zähne (9.6) und die Stirnseite (9.5.1) der Lippe (9.5) auf gleicher axialer Höhe liegen.

16. Set nach einem der vorangehenden Ansprüche, **gekennzeichnet durch** eine Hohlnadel (1) mit einem Stilett und/oder einem Führungsdraht (3).

17. Set nach Anspruch 16, **dadurch gekennzeichnet, dass** Querabmessungen von Stilett und/oder Führungsdraht (3) an das Lumen des Hohlraums der Hohlnadel (1) angepasst sind.

18. Set nach einem der vorangehenden Ansprüche, **gekennzeichnet durch** Dilatoren (6, 7, 8) mit an Führungsrohr (4) und/oder Führungsstab oder aber aneinander angepassten Lumen.

19. Set nach einem der vorangehenden Ansprüche, **gekennzeichnet durch** Arbeitswerkzeuge wie Fräser (10) und Meißel (11), deren radiale Abmessungen insbesondere an das Lumen der Arbeitshülse (9) angepasst sind.

## Claims

1. Instrument set for spinal surgeries, comprising at least one dilator (6, 7, 8), comprising a guide tube (4) and comprising a guide rod (5) which comprises an eccentric cavity, wherein the guide tube (4) can be received in the cavity of the guide rod (5), wherein the guide rod (5) has a lateral slot opening (5.1) connected to the cavity so that the cavity forms a partially cylindrical groove (5.2), and the width (B) of the slot opening (5.1) is smaller than the diameter (D) of the groove (5.2), and wherein the guide rod (5) has a distal tongue (5.5) which is arranged eccentrically with respect to the cavity of the guide rod (5).

2. Set according to claim 1, **characterized in that** the cavity is eccentric with respect to the outer contour (5.3) and/or with respect to the axis of symmetry (S) of the outer contour (5.3) of the guide rod (5).

3. Set according to either claim 1 or claim 2, **characterized in that** the distal tongue (5.5) of the guide rod (5) is arranged eccentrically with respect to an axis of symmetry (S) of the guide rod (5).

4. Set according to any of the preceding claims, **characterized in that** the inner wall surface (5.2.1) of the guide rod (5) forming the cavity (5.2) and surrounding the groove is in the shape of a partial cylindrical casing.

5. Set according to any of the preceding claims, **characterized in that** the outer wall surface (5.3) of the guide rod (5) is in the shape of a partial cylindrical casing.

6. Set according to any of the preceding claims, **characterized in that** the slot opening (5.1) of the groove (5.2) of the guide rod (5) is diagonally opposite the distal tongue (5.5).

7. Set according to any of the preceding claims, **characterized in that** the distal tongue (5.5) of the guide rod (1) has opposing partially arcuate edges (5.6), the distal ends of which lead into a tip (5.4.1) of the distal tongue (5.5).

8. Set according to any of the preceding claims, **characterized by** a working sleeve (9) comprising a longitudinally extending, in particular cylindrical cavity (9.1) which has a cross section that is at least the same as the cross section of the guide tube (4), preferably as the cross section of the guide rod (5).

9. Set according to claim 8, **characterized in that** the working sleeve (9) has a row of teeth (9.4) on its distal end face.

10. Set according to claim 9, **characterized in that** the row of teeth (9.4) extends over a part of the circumference of the working sleeve (9).

11. Set according to claim 10, **characterized in that** the row of teeth (9.4) extends over half of the circumference of the working sleeve (9).

12. Set according to any of claims 9 to 11, **characterized in that** the teeth (9.6) of the row of teeth (9.4) are asymmetrical.

13. Set according to any of claims 9 to 12, **characterized in that** a first flank (9.6.1) of the teeth (9.6) is designed as a flat S inclined toward the distal tip (9.6.3) of a tooth (9.6).

14. Set according to any of claims 9 to 13, **characterized in that** a second flank (9.6.2) is oriented axially parallel.

15. Set according to any of claims 8 to 14, **characterized in that** a distal lip (9.5) extends over a part of the circumference of the working sleeve (9), in particular the tips (9.6.3) of the teeth (9.6) and the end face (9.5.1) of the lip (9.5) being at the same axial height.

16. Set according to any of the preceding claims, **characterized by** a hollow needle (1) comprising a stylet and/or a guide wire (3).

17. Set according to claim 16, **characterized in that** transverse dimensions of the stylet and/or guide wire (3) are adapted to the lumen of the cavity of the hollow needle (1).

18. Set according to any of the preceding claims, **characterized by** dilators (6, 7, 8) comprising lumina adapted to the guide tube (4) and/or guide rod or to one another.

19. Set according to any of the preceding claims, **characterized by** work tools such as milling cutters (10) and chisels (11), the radial dimensions of which are adapted in particular to the lumen of the working sleeve (9).

## Revendications

1. Kit d'instruments pour des opérations rachidiennes comportant au moins un dilatateur (6, 7, 8), comportant un tube de guidage (4) et comportant une tige de guidage (5) comportant une cavité excentrique, dans lequel le tube de guidage (4) peut être reçu dans la cavité de la tige de guidage (5), dans lequel la tige de guidage (5) présente une ouverture en fente (5.1) reliée latéralement à la cavité, de sorte que la cavité forme une rainure (5.2) partiellement cylindrique, et la largeur (B) de l'ouverture en fente (5.1) est inférieure au diamètre (D) de la rainure (5.2), et dans lequel la tige de guidage (5) présente une languette distale (5.5) qui est disposée de manière excentrique par rapport à la cavité de la tige de guidage (5).

2. Kit selon la revendication 1, **caractérisé en ce que** la cavité est réalisée de manière excentrique par rapport au contour extérieur (5.3) et/ou par rapport à l'axe de symétrie (S) du contour extérieur (5.3) de la tige de guidage (5).

3. Kit selon la revendication 1 ou 2, **caractérisé en ce que** la languette distale (5.5) de la tige de guidage (5) est disposée de manière excentrique par rapport à un axe de symétrie (S) de la tige de guidage (5).

4. Kit selon l'une quelconque des revendications précédentes, **caractérisé en ce que** la surface de paroi intérieure (5.2.1) de la tige de guidage (5), laquelle surface entoure la rainure formant la cavité, est en forme d'enveloppe partiellement cylindrique.

5. Kit selon l'une quelconque des revendications précédentes, **caractérisé en ce que** la surface de paroi extérieure (5.3) de la tige de guidage (5) est en forme d'enveloppe partiellement cylindrique.

6. Kit selon l'une quelconque des revendications précédentes, **caractérisé en ce que** l'ouverture en fente (5.1) de la rainure (5.2) de la tige de guidage (5) est diagonalement opposée à la languette distale (5.5).

7. Kit selon l'une quelconque des revendications précédentes, **caractérisé en ce que** la languette distale (5.5) de la tige de guidage (5) présente des bords (5.6) partiellement en forme d'arc et opposés les uns aux autres, dont les extrémités distales débouchent dans une pointe (5.4.1) de la languette distale (5.5).

8. Kit selon l'une quelconque des revendications précédentes, **caractérisé par** un manchon de travail (9) comportant une cavité (9.1) s'étendant longitudinalement et en particulier cylindrique, laquelle présente une section transversale au moins égale à la section transversale du tube de guidage (4), mais de préférence à la section transversale de la tige de guidage (5).

9. Kit selon la revendication 8, **caractérisé en ce que** le manchon de travail (9) présente une rangée de dents (9.4) sur sa face frontale distale.

10. Kit selon la revendication 9, **caractérisé en ce que** la rangée de dents (9.4) s'étend sur une partie de la circonférence du manchon de travail (9).

11. Kit selon la revendication 10, **caractérisé en ce que** la rangée de dents (9.4) s'étend sur la moitié de la circonférence du manchon de travail (9).

12. Kit selon l'une quelconque des revendications 9 à 11, **caractérisé en ce que** les dents (9.6) de la rangée de dents (9.4) sont réalisées de manière asymétrique.

13. Kit selon l'une quelconque des revendications 9 à 12, **caractérisé en ce qu'**un premier flanc (9.6.1) des dents (9.6) est réalisé sous la forme d'un S plat incliné vers la pointe distale (9.6.3) d'une dent (9.6).

14. Kit selon l'une quelconque des revendications 9 à 13, **caractérisé en ce qu'**un second flanc (9.6.2) est orienté parallèlement à l'axe.

15. Kit selon l'une quelconque des revendications 8 à 14, **caractérisé en ce qu'**une lèvre distale (9.5) s'étend sur une partie de la circonférence du manchon de travail (9), dans lequel les pointes (9.6.3) des dents (9.6) et la face frontale (9.5.1) de la lèvre (9.5) sont en particulier à la même hauteur axiale.

16. Kit selon l'une quelconque des revendications précédentes, **caractérisé par** une aiguille creuse (1) comportant un stylet et/ou un fil de guidage (3).

17. Kit selon la revendication 16, **caractérisé en ce que** les dimensions transversales du stylet et/ou du fil de guidage (3) sont adaptées à la lumière de la cavité de l'aiguille creuse (1).

18. Kit selon l'une quelconque des revendications précédentes, **caractérisé par** des dilatateurs (6, 7, 8) comportant des lumières adaptées au tube de guidage (4) et/ou à la tige de guidage ou bien les unes aux autres.

19. Kit selon l'une quelconque des revendications précédentes, **caractérisé par** des outils de travail tels que des fraises (10) et des burins (11), dont les dimensions radiales sont en particulier adaptées à la lumière du manchon de travail (9).
